# EUROPÄISCHE PATENTSCHRIFT

(11) **EP 4 079 240 B1**
(45) Veröffentlichungstag und Bekanntmachung des Hinweises auf die Patenterteilung: **15.11.2023**
(21) Anmeldenummer: 21169197.7
(22) Anmeldetag: 19.04.2021
(51) Int. Cl.: A61B 17/3215, A61B 17/00, A61B 17/295, A61B 18/14

(54) **KLINGENKARTUSCHE FÜR EIN CHIRURGISCHES INSTRUMENT**
BLADE CARTRIDGE FOR A SURGICAL INSTRUMENT
CARTOUCHE À LAMES POUR UN INSTRUMENT CHIRURGICAL

(43) Veröffentlichungstag der Anmeldung: 26.10.2022
(73) Patentinhaber: Erbe Elektromedizin GmbH, 72072 Tübingen (DE)
(72) Erfinder: BOB, Felix, 72108 Rottenburg (DE)
(74) Vertreter: Rüger Abel Patentanwälte PartGmbB

(56) Entgegenhaltungen:
- US-A1- 2010 292 691
- US-A1- 2011 004 208

## Beschreibung

Die Erfindung betrifft eine Messerkartusche für ein chirurgisches Instrument.

Chirurgische Instrumente, insbesondere für den offenchirurgischen Einsatz, sind häufig nach Art von Zangen oder Scheren ausgebildet. Beispielsweise offenbart die US 2011/0004208 A1 ein solches Instrument zum Fusionieren und Trennen von Gefäßen. Die beiden gegeneinander beweglichen Branchen dieses zangenartigen Instruments sind mit Koagulationselektroden versehen. Zusätzlich ist an dem Instrument eine Aufnahme für eine Messerkartusche vorgesehen, die eine Klinge zum Durchtrennen fusionierter Blutgefäße enthält. Die Messerkartusche kann ausgewechselt werden, um das Instrument, wenn die Klinge abgestumpft ist, wieder mit einer scharfen Klinge zu versehen. Außerdem ist die Möglichkeit der Verwendung von Einwegmessern an einem sterilisierbaren oder mit vielfach wiederverwendbaren Instrument eröffnet.

In dem sterilisierbaren Instrument ist zur Führung der Messerklinge einer auswechselbaren Kartusche ein Messerkanal vorgesehen, der bei Gebrauch des Instruments verschmutzen kann und einer Reinigung schwer zugänglich ist. Gefährdungen von Patienten durch im Instrument verbliebenes infektiöses Material müssten jedoch ausgeschlossen werden.

Aus der US 2010/0292691 A1 ist ein Instrument bekannt, bei dem die Versiegelungselektroden und das Messer in einer auswechselbaren Kartusche angeordnet sind.

Es ist Aufgabe der Erfindung, eine Maßnahme anzugeben, wie die Sicherheit für Patient und/oder Personal bei Anwendung entsprechender Instrumente verbessert werden kann.

Diese Aufgabe wird mit der Messerkartusche nach Anspruch 1 gelöst, die mit einem chirurgischen Instrument verbunden werden kann.

Die Messerkartusche kann an einem zangenartigen chirurgischen Instrument eingesetzt werden, das dazu eingerichtet ist, zwischen zwei Branchen gefasstes biologisches Gewebe mittels einer Klinge zu durchtrennen. Die Klinge ist in einem Klingengehäuse verschiebbar gelagert; das Klingengehäuse ist wiederum in dem Kartuschengehäuse verschiebbar gelagert. Dieses Konzept ermöglicht es, zunächst das Klingengehäuse mit der darin geschützt gelagerten Klinge bis an das zwischen den Branchen gefasste Gewebe heranzuschieben, um erst dann die Klinge aus dem Klingengehäuse heraus zu bewegen, um das Gewebe zu durchtrennen. Wird die Klinge nach vollendetem Schnitt in das Klingengehäuse zurückgezogen, kann sie Gewebeanhaftungen mit sich führen und somit das Klingengehäuse innen kontaminieren. Ein in dem Instrument vorgesehener Kanal, durch den das Klingengehäuse aus dem Kartuschengehäuse heraus bis zu den das Gewebe fassenden Branchen geschoben wird, bleibt jedoch sauber. Jedes mit dem Gewebe in Berührung gewesene Teil ist in das Klingengehäuse und mit diesem gegebenenfalls in das Kartuschengehäuse zurückgezogen und wird nach Gebrauch zusammen mit dem Kartuschengehäuse entsorgt. Jedes mit dem Messerkanal des Instruments in Berührung kommende Teil, nämlich das Klingengehäuse, kommt nicht mit dem Gewebe in Berührung. Damit erleichtert die erfindungsgemäße Messerkartusche die Reinigung und Sterilisierung des chirurgischen Instruments. Außerdem wird ein möglicher Verschleiß des Instruments vermindert, weil die scharfe Klinge nicht in der Messernut des Instruments sondern in dem Klingengehäuse geführt und bewegt wird. Die Sicherheit für Patienten ist durch dieses Konzept erhöht.

Das Klingengehäuse kann aus Metall oder auch aus einem Kunststoff oder aus einer Kombination daraus ausgebildet sein. Das Klingengehäuse kann als die Klinge umschließendes Reckteckrohr oder als Schiene ausgebildet und flexibel verformbar ausgebildet sein. So kann es unter flexibler Verformung auch durch eine bogenförmige Nut in dem Instrument bewegt werden.

Die erfindungsgemäße Messerkartusche führt die Klinge in dem Klingengehäuse an ihren Einsatzort, d.h. an das zwischen den Branchen gefasste Gewebe heran. Das Klingengehäuse kann dabei in einer in dem Instrument vorgesehenen Nut z.B. durch den Scharnierbereich des Instruments geschoben werden. Die Klinge und insbesondere deren Schneidkante sind geschützt. Weder kann die Schneidkante an Teilen des Instruments entlang streifen und dadurch abstumpfen, noch kann sie an solchen Instrumententeilen Kratzer oder dergleichen verursachen, in denen sich dann Verschmutzungen halten könnten. Dies erhöht die Patientensicherheit.

Die Messerkartusche ist vorzugsweise mit einem Rastmittel versehen, das eine lösbare Befestigung des Kartuschengehäuses an dem Instrument ermöglicht. Das Rastmittel kann durch ein gesondertes Betätigungselement aus einer Rastposition in eine Löseposition überführt werden. Das Rastmittel kann alternativ auch durch das erste Betätigungselement betätigbar sein, mit dem das Klingengehäuse bewegbar ist. Die Bedienung wird damit einfach und intuitiv.

Das Klingengehäuse weist vorzugsweise eine erste Feststellposition auf, in der das Klingengehäuse vollständig innerhalb des Kartuschengehäuses positioniert ist. Ein an dem Kartuschengehäuse vorgesehener Einsatzfühler kann dazu vorgesehen sein, eine Betätigung des ersten Betätigungselements und/oder eine Bewegung des Klingengehäuses aus der ersten Feststellposition heraus zu blockieren. Der Einsatzfühler dient zur Sperrung der Bewegung des Klingengehäuses, wenn sich die Messerkartusche außerhalb eines Instruments befindet. Der Einsatzfühler dient weiter zur Freigabe einer Bewegung des Klingengehäuses, wenn die Messerkartusche in das Instrument eingesetzt ist.

Der Einsatzfühler ist vorzugsweise ein beweglich gelagertes Sperrglied, das federnd gelagert ist und zwischen einer Sperrposition und einer Freigabeposition beweglich ist. In der Sperrposition blockiert er eine Bewegung des Klingengehäuses aus seiner ersten Feststellposition (Ruheposition) heraus. Der Einsatzfühler ist dabei vorzugsweise so angeordnet, dass er von einer Anlagefläche des Instruments aus seiner Sperrposition gegen die Kraft seiner Feder in eine Freigabeposition gedrängt wird, wenn die Messerkartusche an das Instrument angekuppelt wird. Erst dann ist eine Bewegung des Klingengehäuses freigegeben. Dadurch sind die Klinge und ihr Klingengehäuse, solange die Messerkartusche nicht in das Instrument eingesetzt ist, fest in der Kartusche gehalten. Sie können auch nicht durch ungeschickte Handhabung aus dem Kartuschengehäuse heraus geschoben werden. Verletzungen des handhabenden Personals, Übertragung von Infektionen oder dergleichen, sind dadurch weitgehend ausgeschlossen.

Ist das Kartuschengehäuse an das Instrument angekoppelt und gibt der Einsatzfühler somit die Bewegung des Klingengehäuses frei, kann dieses durch Betätigung des ersten Betätigungselements in eine zweite Feststellposition (Aktivposition) überführt werden, in der das Klingengehäuse aus dem Kartuschengehäuse heraus ragt.

Das Instrument weist typischerweise einen Kanal auf, der von einem proximal zu einem Scherengelenk des Instruments angeordneten Kartuschensitz bis zu dem Messerschlitz reicht, der in den Branchen des Instruments ausgebildet ist. In der zweiten Feststellposition ist das Klingengehäuse bis zu dem Messerschlitz vorgeschoben. Bei der Bewegung des Klingengehäuses aus der ersten Feststellposition in die zweite Feststellposition und zurück ist die Klinge fest (unverschiebbar) mit dem Klingengehäuse gekoppelt. Diese Koppelung kann in der zweiten Feststellposition durch eine entsprechende Freigabebewegung des ersten Betätigungselements beseitigt werden, womit eine axiale Bewegung der Klinge dann auch in Bezug auf das Klingengehäuse freigegeben ist. Die Freigabe erfolgt über eine Übergabeeinrichtung, die beim Trennen des Betätigungselementes von der Klinge zugleich die Klinge antriebsmäßig mit dem zweiten Betätigungselement verbindet. Das zweite Betätigungselement kann nun dazu benutzt werden, die Klinge aus dem Klingengehäuse heraus zu bewegen und dabei beispielsweise biologisches Gewebe zu durchtrennen. Die Übergabeeinrichtung kann auch als "Weiche" bezeichnet werden, die dazu eingerichtet ist, alternativ das erste oder das zweite Betätigungselement mit der Klinge antriebsmäßig zu verbinden. Dabei verbindet die Weiche das erste Betätigungselement zugleich mit dem Klingengehäuse und der Klinge. Ist die Weiche umgeschaltet, verbindet die Weiche das zweite Antriebselement ausschließlich mit der Klinge.

Zwischen dem ersten Betätigungselement und dem zweiten Betätigungselement kann eine Betätigungssperre wirksam sein, die eine Betätigung des zweiten Betätigungselements zur Bewegung der Klinge erst dann freigibt, wenn durch entsprechende Bewegung des ersten Betätigungselements die Übergabeeinrichtung (Weiche) aktiviert wurde, womit die Klinge von dem Klingengehäuse entkoppelt und zugleich das zweite Betätigungselement mit der Klinge gekoppelt wird. Dieses Konzept kann auch bei Ausführungsformen der Messerkartusche ohne Klingengehäuse oder mit einem kurzen Klingengehäuse Anwendung finden, das gänzlich in der Messerkartusche verbleibt, oder aus dem die Klinge schon herausragt oder austritt, während es noch distal bewegt wird.

Dem ersten Betätigungselement kann eine Verschiebesperre zugeordnet sein, die eine Verschiebung und somit eine Verlagerung des Klingengehäuses in dem Kartuschengehäuse sperrt, solange die Übergabeeinrichtung eine Antriebsverbindung zwischen dem zweiten Betätigungselement und der Klinge zulässt und keine Antriebsverbindung zwischen dem ersten Betätigungselement und der Klinge besteht.

Es ist außerdem möglich, das erste Betätigungselement zum Lösen der Rastverbindung zwischen der Messerkartusche und einem Instrument zu nutzen. Dazu kann das an dem Kartuschengehäuse vorgesehene Rastmittel einen Nocken aufweisen, mit dem ein Rastglied gegen die Kraft einer Feder in Löserichtung bewegbar ist. Auch kann das erste Betätigungselement mit einem Nocken versehen sein, der durch entsprechende Kraftbeaufschlagung des ersten Betätigungselements gegen den Nocken des Rastglieds bewegbar ist.

Mit dem so beschriebenen Mechanismus ist sichergestellt, dass die Klinge erst dann aus dem Kartuschengehäuse und dem Klingengehäuse herausgeführt werden kann, wenn die Messerkartusche korrekt mit dem Instrument verbunden und das Klingengehäuse in Aktivposition überführt ist. In Aktivposition steht das Klingengehäuse stirnseitig am Einsatzort der Klinge. Die Distanz zwischen dem Kartuschengehäuse und dem Einsatzort der Klinge wird durch das Klingengehäuse überbrückt. Der Einsatzort der Klinge liegt distal bezüglich eines Scharniergelenks des Instruments, während die Messerkartusche bezüglich dieses Gelenks proximal angeordnet ist. Der zur Aufnahme des Klingengehäuses in dem Instrument vorgesehene Kanal wird durch die vorgeschlagene Bauart wenig oder nicht kontaminiert.

Das Kartuschengehäuse kann erst dann von dem Instrument entfernt werden, wenn sowohl die Klinge in das Klingengehäuse eingezogen als auch das Klingengehäuse in das Kartuschengehäuse eingezogen ist. Eine Entriegelung des Kartuschengehäuses von dem Instrument, d.h. eine Lösung von diesem, ist erst möglich, wenn das Klingengehäuse vollständig in das Kartuschengehäuse eingezogen ist. Nach dem Lösen des Kartuschengehäuses von dem Instrument können weder das Klingengehäuse noch die Klinge aus dem Kartuschengehäuse herausgeschoben werden. Das Klingengehäuse und die Klinge sind in dem Kartuschengehäuse arretiert. Die Schneidkante der Klinge bleibt jedenfalls unzugänglich in der Messerkartusche aufgenommen.

Weitere vorteilhafte Einzelheiten ergeben sich aus Ansprüchen sowie aus der Zeichnung mit ihren Figuren und der zugehörigen Beschreibung. Es zeigen:
Figur 1 ein Instrument mit einer erfindungsgemäßen Messerkartusche in Seitenansicht in Prinzipdarstellung,
Figur 2 die Messerkartusche nach Figur 1 in gestrichelter Darstellung in handhabbarem Zustand, in Seitenansicht,
Figur 3 die Messerkartusche nach Figur 2 mit ihrem in Aktivposition befindlichen Klingengehäuse und einer herausschiebbaren Klinge (herausgeschoben in gestrichelter Darstellung)
Figur 4 die Messerkartusche nach Figur 3, geschnitten entlang der Linie IV-IV,
Figur 5 einen Abschnitt des Instruments nach Figur 1 ohne Messerkartusche, in stark vergrößerter schematisierter perspektivischer Darstellung,
Figur 6 die Messerkartusche nach Figur 3, in ausschnittsweiser perspektivischer und stark verkürzter Darstellung,
Figur 7 bis 11 die Messerkartusche nach Figur 2 und 3 in verschiedenen Positionen und Zuständen, in Darstellung des kinematischen Prinzips.

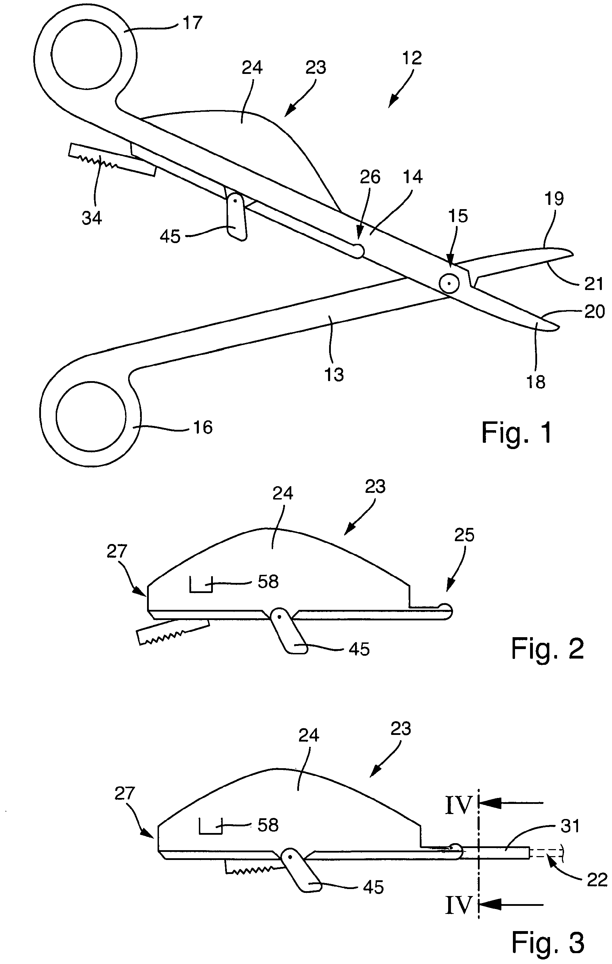

In Figur 1 ist ein chirurgisches Instrument 12 mit zwei Schenkeln 13, 14 veranschaulicht, die nach Art einer Zange oder Schere über ein Gelenk 15 schwenkbar miteinander verbunden sind. An ihren proximalen Enden weisen die Schenkel 13, 14 Handgriffe 16, 17 auf. An ihren distalen Enden sind die Schenkel 13, 14 Branchen 18, 19, zwischen denen biologisches Gewebe gefasst und zusammengedrückt werden kann. Beispielsweise kann es sich bei dem Gewebe um Blutgefäße, andere Hohlgefäße oder auch Sehnen, Haut, Muskeln oder dergleichen handeln. Die Branchen 18, 19 können glatt oder leicht profiliert ausgebildet sein. Sie können außerdem aus einem elektrisch leitenden oder elektrisch nicht leitenden Material ausgebildet sein. Sind die aufeinander zuweisenden Flächen 20, 21 der Branchen 18, 19 elektrisch leitend ausgebildet, können sie beispielsweise auch als Elektrode ausgebildet sein, um zwischen den Branchen 18, 19 gefasstes Gewebe einem Stromfluss auszusetzen und zu koagulieren und/oder zu fusionieren. Die aufeinander zu weisenden Flächen 20, 21 bilden dann Elektroden, denen über ein nicht weiter veranschaulichtes Kabel oder auch eine am Instrument 12 vorgesehene Spannungsquelle Strom zugeführt wird. Außerdem können weitere Elemente, wie Schalter oder dergleichen vorgesehen sein, um den Stromfluss zu steuern, insbesondere ein und auszuschalten

Das Instrument 12 ist vorzugsweise ein Koagulations- und Dissektionsinstrument, das zur Dissektion von Gewebe eine Klinge (Figur 3, Figur 4, Figur 6) nutzt. Die Klinge 22 gehört zu einer Messerkartusche 23, die an dem Instrument 12 lösbar gehalten ist. Beispielsweise weist der Schenkel 14 dazu eine z.B. als Aufnahmefach ausgebildeten Sitz auf, in den die Messerkartusche 23 einsetzbar ist. Die Messerkartusche 23 ist vorzugsweise zum einmaligen Einsatz vorgesehen, während das Instrument 12 als sterilisierbares vielfach wiederverwendbares Instrument 12 ausgebildet ist. Während die Messerkartusche 23 nach Benutzung von dem Instrument 12 getrennt und entsorgt wird, wird das Instrument 12 einer Reinigung und Sterilisation zugeführt.

Die Messerkartusche 23 weist ein Kartuschengehäuse 24 auf, das zum Beispiel als Kunststoffspritzgussteil ausgebildet sein kann. An seinem in Gebrauch distalen Ende ist es mit einer Koppelstruktur 25 versehen, die in eine entsprechende Komplementärstruktur des Schenkels 14 passt. Beispielsweise kann die Koppelstruktur ein wulstartiger Vorsprung sein, der in eine entsprechend geformte Quernut 26 des Instruments 12 passt. An seinem entgegen gesetzten proximalen Ende kann das Kartuschengehäuse 24 ein Rastmittel 27 aufweisen, zu dem ein in dem Kartuschengehäuse 24 angeordnetes und beispielsweise schwenkbar gelagertes Rastglied 28 gehören kann. Das Rastglied 28 kann mit einem Rastvorsprung 29 des Instruments 12 zusammenwirken, der in das Rastglied 28 einrastet sobald das Kartuschengehäuse 24 durch Schwenken um eine von der Quernut 26 definierte Querachse an den Schenkel 14 herangeführt wird. Die Koppelstruktur 25 und die Quernut 26 bilden dabei ein Schwenkscharnier. Zu dem Rastmittel 27 kann außerdem eine Feder 29 beispielsweise in Gestalt einer Druckfeder, Biegefeder, Blattfeder oder dergleichen gehören. Das Federmittel 29 kann einteiliger Bestandteil des Rastglieds 28 oder eine an dem Kartuschengehäuse 24 innen ausgebildete federnde Zunge oder auch ein gesondertes Federelement sein. Das Rastglied 28 ist in dem Kartuschengehäuse 24 durch ein Schwenklager 30 beweglich, zum Beispiel verschiebbar oder auch schwenkbar gehalten.

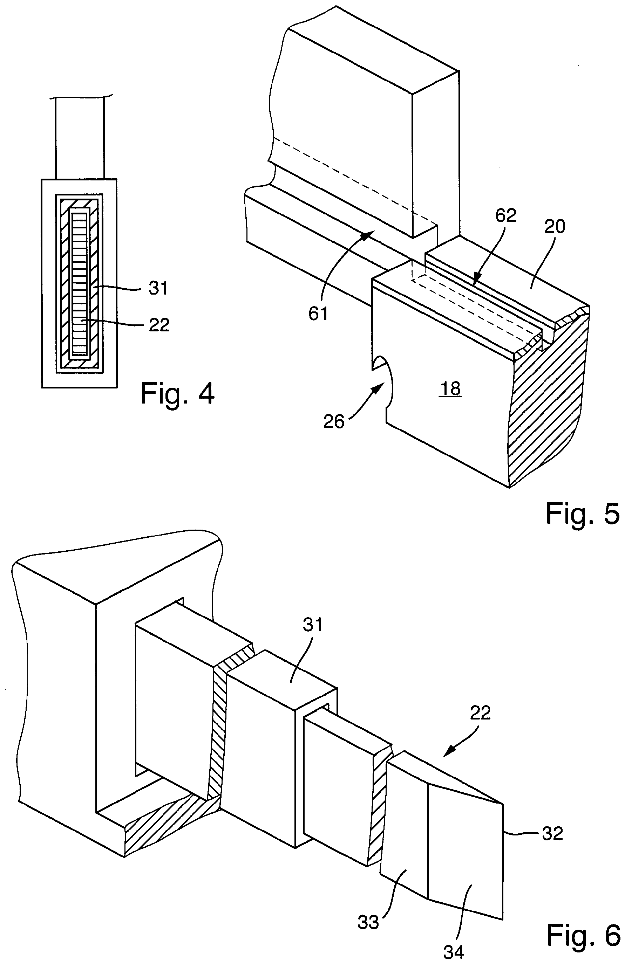

In dem Kartuschengehäuse 24 ist außerdem ein Klingengehäuse 31 angeordnet, das als flaches Rechteckrohr oder auch als flache Führungsschiene vorzugsweise aus Metall oder aber auch aus Kunststoff ausgebildet sein kann. Es wird dazu insbesondere auf die Figuren 4 und 6 verwiesen, die veranschaulichen, dass das Klingengehäuse 31 zumindest in seinem vorderen, aus dem Kartuschengehäuse herausschiebbaren Teil die flache Klinge 22 sowohl an den beiden Flachseiten als auch an der oberen und unteren Schmalseite umgibt. Das Klingengehäuse 31 kann jedoch auch zumindest auf einer Teillänge, insbesondere seinem proximalen, immer in dem Kartuschengehäuse 24 verbleibenden Teil an der oberen Schmalseite offen sein. Dort kann ein Fortsatz 22a der Klinge 22 aus dem Klingengehüuse 31 heraus ragen.

Das Klingengehäuse 31 kann abschnittsweise auch an einer der Flachseiten offen sein. Das Klingengehäuse 31 kann z.B. an einer Seite offen sein, die einer Schneidkante 32 der Klinge 22 abgewandt ist. Wie Figur 6 veranschaulicht, kann die Schneidkante 32 bezüglich der Klinge 22 außermittig angeordnet sein und unmittelbar an eine ebene Seitenfläche der Klinge 22 grenzen. Die andere Seitenfläche 33 geht dann in eine Schrägfläche 34 über die sich bis zu der Schneidkante 32 erstreckt. Vorzugsweise ist das Klingengehäuse 31 so lang ausgebildet, dass die Klinge 22 in zuzrückgezogener Position vollständig in dem Klingengehäuse 31 geborgen ist. Die Schneidkante 32 steht dabei innerhalb des Klingengehäuses 31. Es ist aber auch möglich, das Klingengehäuse verkürzt auszubilden, so dass die Klinge 22 immer etwas aus dem Klingengehäuse heraus ragt und die Schneidkante 32 vor dem distalen Ende des Klingengehäuses 31 steht. Weiter existieren Ausführungsformen ohne Klingengehäuse.

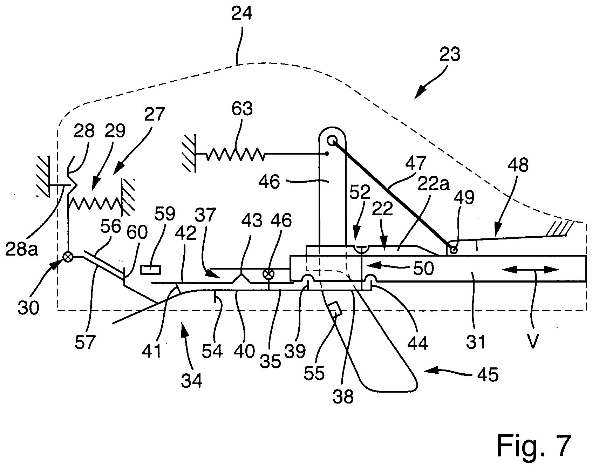

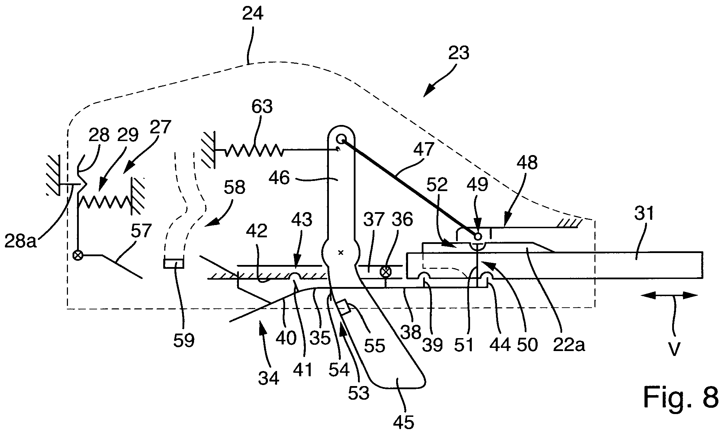

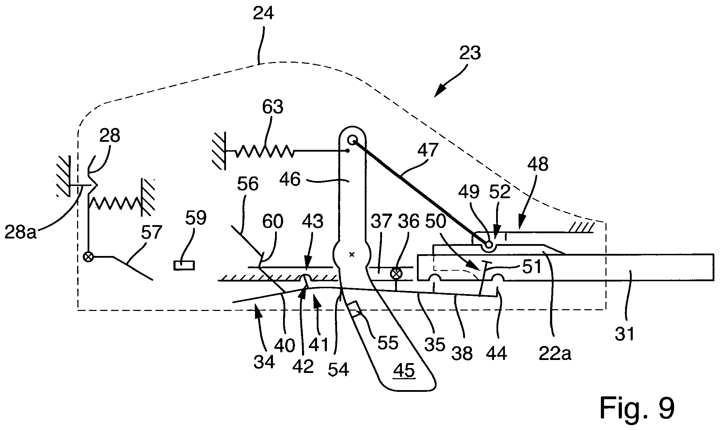

Das Klingengehäuse 31 ist in dem Kartuschengehäuse 24 prinzipiell verschiebbar angeordnet und in einer Verschieberichtung V (Figur 7, Figur 8) geführt. Dabei lässt sich das Klingengehäuse 31 aus einer ersten Feststellposition gemäß Figur 7 in eine zweite Feststellposition gemäß Figur 9 überführen. In der ersten Feststellposition ist das Klingengehäuse 31 von dem Kartuschengehäuse 24 vollständig aufgenommen. In der zweiten Feststellposition nach Figur 8 und Figur 9 (Aktivposition) ragt das Klingengehäuse 31 aus dem distalen Ende des Kartuschengehäuses 24 heraus.

Zur Bewegung des Klingengehäuses 31 aus der ersten Feststellposition in die zweite Feststellposition gemäß Figur 8 und 9 dient ein erstes Betätigungselement 34, zu dem eine Schwenk- und Sperrwippe 35 gehört. Diese Schwenk- und Sperrwippe 35 weist einen Drehzapfen 36 auf, der in einer sich in Verschieberichtung V erstreckenden Nut 37 verschiebbar geführt und zugleich drehbar gelagert ist. Die Schwenk- und Sperrwippe 35 bildet einen zweiarmigen Hebel, dessen erster Arm 38 mit dem Klingengehäuse 31 verbunden ist, um eine Bewegung in Verschieberichtung V auf das Klingengehäuse 31 zu übertragen. Dazu kann der Arm 38 mit einem Vorsprung 39 versehen sein, der in einer Ausnehmung des Klingengehäuses 31 greift.

Die Schwenk- und Sperrwippe 35 weist einen zweiten Arm 40 auf, an dem das erste Betätigungselement 34 gehalten sein kann. Das Betätigungselement 34 ist vorzugsweise fest mit der Schwenk- und Sperrwippe 35 verbunden. Außerdem ist einer der Arme 38, 40, vorzugsweise der Arm 40, mit einer Schwenk- und Verschiebesperre 41 versehen, die zum Beispiel als Nocken oder Vorsprung ausgebildet ist. Dieser gleitet bei Verschiebung der Schwenk- und Sperrwippe 35 in Verschieberichtung V an einer Sperrfläche 42 entlang, die so angeordnet ist, dass sie ein Schwenken der Schwenk- und Sperrwippe 35 blockiert. Die Sperrfläche 42 weist jedoch eine Ausnehmung 43 auf, die der Schwenk- und Verschiebesperre 41 gegenüber liegt, wenn die Schwenk- und Sperrwippe 35 in ihrer zweiten Feststellposition, d.h. den distalen maximal weit ausgeschobener Position (Aktivposition) steht, wie es die Figuren 8 und 9 veranschaulichen.

Der erste Arm 38 trägt außerdem eine Kupplung 44, die durch eine Ausnehmung des Klingengehäuses 31 eine in Verschieberichtung V feste Verbindung zwischen dem ersten Arm 38 und der Klinge 22 herstellt. Die Klinge 22 weist dazu an ihrer der Kupplung 44 zugewandten Stelle eine Ausnehmung auf, in die ein Vorsprung des Arms 38 greift, welcher Vorsprung die Kupplung 44 bildet. Somit ist die Klinge 22 vermittels des Vorsprungs 39 und der Kupplung 44 in Verschiebrichtung V fest an das Klingengehäuse 31 gekuppelt, solange die Schwenk- und Sperrwippe 35 in nicht verschwenkter Position steht, d.h. solange die Schwenk- und Verschiebesperre 41 nicht in die Ausnehmung 43 greift (Figur 7, 8 und 11).

Zur Betätigung der Klinge 22, d.h. zum Ausfahren aus dem Klingengehäuse 31, ist ein zweites Betätigungselement 45 vorgesehen, das vorzugsweise durch einen an dem Kartuschengehäuse 24 schwenkbar gelagerten zweiarmigen Hebel gebildet ist. Die Schwenkachse des zweiten Betätigungselements 45 ist vorzugsweise quer zu der Verschiebrichtung V orientiert. Das zweite Betätigungselement 45 ragt dabei, wie insbesondere die Figuren 1 bis 3 zeigen, von dem Kartuschengehäuse 24 weg. Ist die Messerkartusche 23 indes mit dem Schenkel 14 des Instruments 12 verbunden, ragt das Betätigungselement 45 in Richtung auf den anderen Schenkel 13 und geht bei geschlossenem Instrument 12 infolge seitlichen Versatzes an diesem vorbei.

Ein in dem Kartuschengehäuse 24 angeordneter Hebelarm 46 des zweiten Betätigungselements 45 kann über einen Pleuel 47 mit der Klinge 22 gekoppelt werden. Dazu weist die Klinge 22 an ihrem proximalen Ende einen aus dem Klingengehäuse 31 herausragenden Fortsatz 22a auf, der an seinem distalen Ende als Rampe ausgebildet ist. Auf diesem Fortsatz 22a ruht ein Riegel 48, der durch einen federnden, an dem Kartuschengehäuse 24 gehaltenen Arm gebildet sein kann, der ein gegabeltes Ende aufweist. In dem gegabelten Ende des Riegels 48 ist ein Kupplungszapfen 49 gefangen, der an einem Ende des Pleuels 47 angeordnet ist.

Die Schwenk- und Sperrwippe 35 trägt an ihrem distalen Ende des Arms 38 einen Übergabemechanismus 50. Dieser wird durch einen Fortsatz 41 des Arms 38 gebildet, der sich von dem Arm 38 ausgehend an dem Klingengehäuse 31 vorbei neben eine in dem Fortsatz 22a ausgebildete Kupplungsausnehmung 52 erstreckt. Der Fortsatz lässt den Kupplungszapfen 50 nicht in die Kupplungsausnehmung 52 eindringen, solange die Schwenk- und Sperrwippe 35 in nicht verschwenkter Stellung steht, d.h. die Schwenk- und Verschiebesperre 41 nicht in die Ausnehmung 43 fasst. Wird der Hebelarm 38 der Schwenk- und Sperrwippe 35 nach oben geschwenkt (Figur 8), greift die Kupplung 44 in das Klingengehäuse und der Fortsatz 51 dränget den Kupplungszapfen 49 aus der Ausnehmung 52. Schwenkt der Hebelarm 38 nach unten (Figur 9) rastet der Kupplungszapfen 49 in die Ausnehmung 52 ein. So stellt der Übergabemechanismus 50 sicher, dass die Klinge 22 entweder mit dem ersten Betätigungselement 34 und dem Klingengehäuse 31 oder mit dem zweiten Betätigungselement 45 gekoppelt ist.

Außerdem ist zwischen der Schwenk- und Sperrwippe 35 und dem zweiten Betätigungselement 45 eine Betätigungssperre 53 (siehe Figur 8) wirksam, die ein Betätigen des zweiten Betätigungselements 45 temporär sperrt. Die Sperre ist wirksam, wenn das Klingengehäuse 31 in expandierter Position, d.h. in Aktivposition steht. Damit ist die Sperre solange wirksam, wie die Schwenk- und Sperrwippe 35 noch mit der Klinge 22 gekuppelt ist. Ein entsprechender Vorsprung 54 der Schenk- und Sperrwippe 35 gibt den Weg für einen Vorsprung 55 des zweiten Betätigungselements 45 erst dann frei, wenn die Wippe 35 gekippt ist, sodass die Schwenk- und Verschiebesperre 41 in die Ausnehmung 43 greift und die Kupplung 44 gelöst und die Klinge 22 freigegeben ist. Dieser Zustand ist in Figur 9 veranschaulicht. Das Pleuel 47 stellt nun eine Antriebsverbindung zwischen dem zweiten Betätigungselement 45 und der Klinge 22 her, die dann durch Betätigung des zweiten Betätigungselements 45 aus dem Klingengehäuse 31 herausgeschoben werden kann.

Der zweite Arm 40 ist außerdem mit einem Keil oder Nocken 56 verbunden, der mit einem Keil oder Nocken 57 des Rastglieds 28 verbunden ist. Die Nocken 56 und 57 sind so positioniert, dass sie in Eingriff gelangen, wenn die Schwenk- und Sperrwippe 35 in ihrer proximalen Extremposition steht.

Das Kartuschengehäuse 24 kann außerdem einen Einsatzfühler 58 aufweisen, der durch eine an dem Kartuschengehäuse 24 ausgebildete, etwas über die Außenkontur desselben hervor stehende federnde Zunge gebildet sein kann. Diese federnde Zunge weist ein unteres Ende auf, das quer zu der Verschieberichtung V beweglich ist. Dieses untere Ende kann als Sperrklotz 59 ausgebildet sein, dem eine an dem Hebelarm 40 ausgebildete Anschlagfläche zugeordnet ist. Diese ist so angeordnet, dass ein wesentliches Verschieben der Schwenk- und Sperrwippe 35 aus der proximalen Position heraus nicht möglich ist, solange der Einsatzfühler 58 nicht aus seiner Ruheposition herausgedrängt ist.

Die insoweit beschriebene Messerkartusche 23 arbeitet wie folgt:

Figur 7 veranschaulicht die Messerkartusche 23 im Auslieferungszustand, in der sie sicher handhabbar ist. Die Klinge 22 ist vollständig in das Klingengehäuse 31 eingezogen und darin gesichert. Das Klingengehäuse 31 ist vollständig in dem Kartuschengehäuse 24 positioniert und darin arretiert. Das erste Bedienelement 34 steht in proximaler Position. Eine Betätigung des zweiten Betätigungselements 45 führt zu keiner Veränderung dieser Situation. Weder ist die Klinge 22 aus dem Klingengehäuse 31 noch das Klingengehäuse 31 aus dem Kartuschengehäuse 24 herausschiebbar.

Die Messerkartusche 23 kann an das Instrument 12 angeschlossen werden, wobei das Rastglied 28 mit einem entsprechenden Rastvorsprung 28a in Eingriff kommt, der an dem Instrument 12 ausgebildet sein kann. Zugleich drängt ein Element des Instruments 12, beispielsweise eine an dem Schenkel 14 vorgesehene Fläche den Einsatzfühler 58 entgegen seiner Federwirkung etwas in Richtung des Inneren des Kartuschengehäuses 24. Sofern der Einsatzfühler 58 über die Kontur des Kartuschengehäuses 24 vorsteht, wird er beispielsweise in die Kontur des Kartuschengehäuses 24 gedrängt. Der Sperrklotz 59 gibt dadurch eine Bewegung des ersten Betätigungselements 34 in Distalrichtung (Figur 7 nach rechts) frei. Der Bediener kann nun das erste Bedienelement 34 in Distalrichtung verschieben. Dabei werden das Klingengehäuse 31 und die in ihm arretierte Klinge 22 in Verschieberichtung V distal aus dem Kartuschengehäuse 24 herausgeschoben wie es Figur 8 veranschaulicht. Das Klingengehäuse 31 dringt dabei in einem dafür vorgesehenen Kanal 61 (Figur 5) bis zu der Branche 18 vor.

Der Kanal 61 kann sich durch den Bereich des Scharniers 15 hindurch erstrecken. Er endet an der Branche 18 an einem Messerschlitz 62 und stößt vorzugsweise mit seiner Stirnfläche an dem Eingang des Messerschlitzese 62 an. Bei dem Vorschieben des Klingengehäuses 31 in dem Kanal 61 ist die Schneidkante 32 der Klinge 22 von dem Klingengehäuse 31 aufgenommen und geschützt.

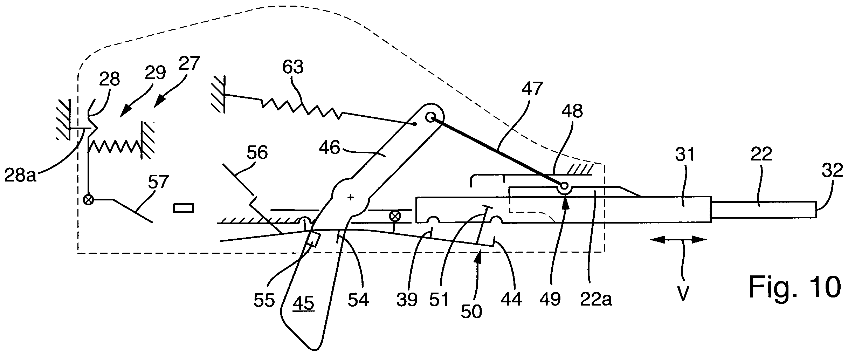

Die Schwenk- und Sperrwippe 35 arretiert die Klinge 22 noch immer in dem Klingengehäuse 31 während das Bedienelement 34 in distaler Richtung bewegt wird. Während dieses Vorgangs ist ein Entkoppeln von Klingengehäuse 31 und Klinge 22 nicht möglich, weil die Schwenk- und Verschiebesperre 41 an der Sperrfläche 42 entlang läuft und ein Schwenken der Schwenk- und Sperrwippe 35 verhindert. Erst in der maximal distalen Position, d.h. in der zweiten Feststellposition des Klingengehäuses 31, steht die Schwenk- und Verschiebesperre 41 vor der Ausnehmung 43, wie es Figur 8 veranschaulicht. Nun ist eine Schwenkbewegung der Schenk- und Sperrwippe 35 freigegeben. Der Bediener kann jetzt das erste Betätigungselement 34 an das Kartuschengehäuse 24 herandrücken und dadurch die Schwenk- und Sperrwippe 35 etwas schwenken. Wie aus Figur 9 ersichtlich, verlässt die Kupplung 44 dabei das Klingengehäuse 31 und koppelt sich dabei auch von der Klinge 22 ab. Zugleich gibt der Übergabemechanismus 50 die Kupplungsausnehmung 52 frei, sodass der Kupplungszapfen 49 in die Kupplungsausnehmung 52 der Klinge 22 eindringen kann. Auch gibt der Vorsprung 54 den Vorsprung 55 frei, sodass das zweite Betätigungselement 45 nun betätigt, d.h. geschwenkt werden kann, wie es Figur 10 veranschaulicht.

Wird das zweite Betätigungselement 45 betätigt (in den Figuren in Uhrzeigerrichtung geschwenkt) schiebt es mit seinem Pleuel 47 die Klinge 22 in Verschieberichtung V, d.h. distal aus dem Klingengehäuse 31 heraus. Die Klinge 22 dringt in den Messerschlitz 62 ein und die Schneidkante 32 läuft in dem Messerschlitz 62 (Figur 5) entlang, Die Klinge 22 kann zum Beispiel zwischen den Branchen 18, 19 gefasstes Gewebe durchtrennen. Der Messerschlitz kann dabei gerade oder, wenn die Klinge 22 ausreichend flexibel ist, auch gekrümmt sein. Die Klinge 22 ist dabei vorzugsweise so gestaltet, dass die Schneidkante 32 auf der Seite der konvexen Flanke des Messerschlitzes 62 angeordnet ist.

Wird das zweite Betätigungselement 45 freigegeben, zieht eine entsprechende Rückholfeder 63 das Betätigungselement 45 in seine Ruheposition zurück. Damit wird zugleich über das Pleuel 47 die Klinge 22 zurück in das Klingengehäuse 31 gezogen. Es wird so, ausgehend von dem Zustand nach Figur 10, wieder der Zustand nach Figur 9 erreicht. Das Instrument 12 und die Messerkartusche 23 bleiben jedoch arbeitsbereit. Das Betätigungselement 45 kann wiederholt betätigt werden, worauf die Klinge 22 immer wieder aus- und einfährt.

Soll die Messerkartusche 23 von dem Instrument 12 entfernt werden, wird zunächst das zweite Betätigungselement 45 freigegeben, sodass der Zustand nach Figur 9 hergestellt ist. Danach wird das erste Betätigungselement 34 von dem Kartuschengehäuse 24 weg nach unten so geschwenkt, dass die Kupplung 44 in das Klingengehäuse 31 eingreift. Somit ist die Klinge 22 in dem noch expandierten Klingengehäuse 31 fest arretiert. Eine Betätigung des zweiten Betätigungselements ist nun nicht mehr möglich, weil der an der Schwenk- und Sperrwippe 35 vorgesehene Vorsprung 54 dem an dem zweiten Betätigungselement 55 vorgesehenen Vorsprung 55 den Weg versperrt. Der Übergabemechanismus 50 hebt dabei den Zapfen 49 des Pleuels 47 aus der Kupplungsausnehmung 52 heraus. Dadurch ist das zweite Betätigungselement 45 nun von der Klinge 22 abgekoppelt.

Das erste Betätigungselement 34 wird danach in Proximalrichtung bewegt, d.h. in Figur 8 nach rechts, sodass es die Position nach Figur 7 erreicht. Der Zapfen 49 des Pleuels 47 verlässt den Fortsatz 22a der Klinge 22. Wegen der Schwenk- und Verschiebesperre 41, die an der Sperrfläche 42 entlang läuft, kann die Schwenk- und Sperrwippe 35 dabei nicht geschwenkt werden, sodass die feste Kopplung zwischen der Klinge 22 und dem Klingengehäuse 31 nicht gelöst werden kann.

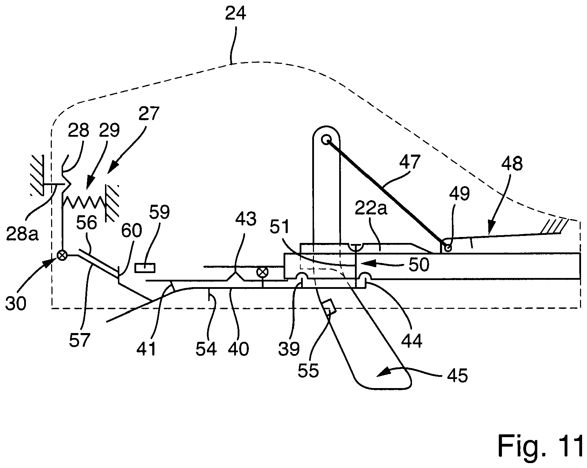

Soll nun die Messerkartusche 23 vollends von dem Instrument 12 gelöst werden, wird das erste Betätigungselement 34 über seine in Figur 7 veranschaulichte proximale Endposition hinaus in Proximalrichtung gedrängt, wie es Figur 11 veranschaulicht. Dabei läuft der Nocken 56 an den Nocken 57 an, sodass das Sperrglied 28 gegen die Kraft der Feder 29 verschwenkt wird. Das Rastmittel 27 ist dadurch gelöst und die Messerkartusche 23 kann von dem Instrument 12 abgenommen werden.

Bei allen Ausführungsformen von Messerkartuschen 23 ohne Klingengehäuse 31 gilt die vorige Beschreibung entsprechend, jedoch mit der Maßgabe, dass das Klingengehäuse 31 und optional auch der Vorsprung 39 entfallen.

Eine erfindungsgemäße Messerkartusche 23 ist zum Einsatz an einem chirurgischen Instrument 12 vorgesehen. Die Messerkartusche 23 weist eine Klinge auf, die über ein zweites Betätigungselement 45 betätigt werden kann. Die Klinge 22 ist in einem Klingengehäuse 31 gehalten, das über ein erstes Betätigungselement 34 von einer ersten Feststellposition (Passivposition) in eine zweite Feststellposition (Aktivposition) überführt werden kann. In der Passivposition ist das Klingengehäuse 31 vollständig von dem Kartuschengehäuse 24 aufgenommen und die Klinge 22 ist darin arretiert. In der Aktivposition ragt das Klingengehäuse 31 aus dem Kartuschengehäuse heraus. Erst in Aktivposition kann eine Verriegelung zwischen dem Klingengehäuse 31 und der Klinge 22 gelöst werden, wobei auch erst dann eine Antriebsverbindung zwischen dem zweiten Betätigungselement und der Klinge 22 hergestellt wird.

Durch die Fassung der Klinge 22 in dem Klingengehäuse 31 werden Gewebereste, die beim Rückzug der Klinge von dem Gewebe mitgenommen werden, in dem Klingengehäuse 31 aufgenommen und darin eingeschlossen. Die Reinigung und Wiederverwendung des Instruments 12 ist dadurch einfacher und sicherer.

### Bezugszeichen:

- 12: Instrument
- 13, 14: Schenkel
- 15: Gelenk
- 16, 17: Handgriffe
- 18, 19: Branchen
- 20, 21: aufeinander zu weisende Flächen der Branchen 18, 19
- 22: Klinge
- 22a: Fortsatz
- 23: Messerkartusche
- 24: Kartuschengehäuse
- 25: Koppelstruktur
- 26: Quernut
- 27: Rastmittel
- 28: Rastglied
- 29: Federmittel
- 30: Lager
- 31: Klingengehäuse
- 32: Schneidkante
- 33: Seitenfläche
- V: Verschieberichtung
- 34: erstes Betätigungselement
- 35: Schwenk- und Sperrwippe
- 36: Drehzapfen
- 37: Nut
- 38: erster Arm der Schwenk- und Sperrwippe 35
- 39: Vorsprung
- 40: zweiter Arm der Schwenk- und Sperrwippe 35
- 41: Schwenk- und Verschiebesperre
- 42: Sperrfläche
- 43: Ausnehmung
- 44: Kupplung
- 45: zweites Betätigungselement
- 46: Hebelarm
- 47: Pleuel
- 48: Riegel
- 49: Kupplungszapfen
- 50: Übergabemechanismus
- 51: Fortsatz des Hebelarms 38
- 52: Kupplungsausnehmung
- 53: Betätigungssperre
- 54: Vorsprung der Schwenk- und Sperrwippe 35
- 55: Vorsprung des zweiten Betätigungselements 45
- 56: Nocken der Schwenk- und Sperrwippe
- 57: Nocken des Rastglieds 28
- 58: Einsatzfühler
- 59: Sperrklotz
- 60: Anschlagfläche
- 61: Kanal
- 62: Messerschlitz
- 63: Rückholfeder

## Patentansprüche

1. Messerkartusche (23) für ein chirurgisches Instrument (12),
mit einem Kartuschengehäuse (24), das mit einem Instrument (12) verbindbar ausgebildet ist, **gekennzeichnet durch**
ein Klingengehäuse (31), das in dem Kartuschengehäuse (24) verschiebbar gelagert ist,
eine Klinge (22), die in dem Klingengehäuse (31) verschiebbar gelagert ist,
ein ersten Betätigungselement (34) zur Verschiebung des Klingengehäuses (31) und
ein zweites Betätigungselement (45) zur Verschiebung der Klinge (22) in dem Klingengehäuse (31).

2. Messerkartusche nach Anspruch 1, **dadurch gekennzeichnet, dass** Kartuschengehäuse (24) ein Rastmittel (27) zur lösbaren Verbindung des Kartuschengehäuses (24) mit dem Instrument (12) aufweist.

3. Messerkartusche nach Anspruch 2, **dadurch gekennzeichnet, dass** das Rastmittel (27) durch das erste Betätigungselement (34) betätigbar ist.

4. Messerkartusche nach einem der vorstehenden Ansprüche, **dadurch gekennzeichnet, dass** das Klingengehäuse (31) eine erste Feststellposition aufweist, in der das Klingengehäuse (31) vollständig innerhalb des Kartuschengehäuses (24) positioniert ist.

5. Messerkartusche nach einem der vorstehenden Ansprüche, **dadurch gekennzeichnet, dass** das Klingengehäuse (31) eine zweiten Feststellposition aufweist, in der Klingengehäuse (31) teilweise aus dem Kartuschengehäuse (24) herausragend gehalten ist.

6. Messerkartusche nach einem der vorstehenden Ansprüche, **dadurch gekennzeichnet, dass** die Klinge (22) eine Rückzugsposition aufweist, in der die Klinge (22) vollständig innerhalb des Klingengehäuses (31) positioniert und in dem Klingengehäuse (31) arretiert ist.

7. Messerkartusche nach einem der vorstehenden Ansprüche, **dadurch gekennzeichnet, dass** die Klinge (22) einen Fortsatz (22a) aufweist, der aus dem Klingengehäuse (31) herausschiebbar ist.

8. Messerkartusche nach einem der vorstehenden Ansprüche, **dadurch gekennzeichnet, dass** das erste Betätigungselement (34) an dem Kartuschengehäuse (24) verschiebbar gelagert ist.

9. Messerkartusche nach einem der vorstehenden Ansprüche, **dadurch gekennzeichnet, dass** das erste Betätigungselement (34) an dem Kartuschengehäuse (24) entlang eines vorgegebenen Verschiebwegs gelagert ist.

10. Messerkartusche nach Anspruch 8, **dadurch gekennzeichnet, dass** zwischen dem ersten Betätigungselement (34) und dem Kartuschengehäuse (24) eine Schwenksperre (41) wirksam ist, die ein Schwenken des ersten Betätigungselements (34) nur in einer Endposition des Verschiebewegs zulassend ausgebildet ist.

11. Messerkartusche nach einem der vorstehenden Ansprüche, **dadurch gekennzeichnet, dass** das erste Betätigungselement (34) eine Kupplung (39) zur Verbindung mit dem Klingengehäuse (31) aufweist.

12. Messerkartusche nach einem der vorstehenden Ansprüche, **dadurch gekennzeichnet, dass** das erste Betätigungselement (34) und das zweite Betätigungselement (45) über einen Übergabemechanismus (50) wechselweise mit der Klinge (22) kuppelbar sind.

13. Messerkartusche nach einem der vorstehenden Ansprüche, **dadurch gekennzeichnet, dass** das zweite Betätigungselement (45) durch Schwenken des ersten Betätigungselements (35) mit der Klinge (22) operativ kuppelbar ist.

14. Messerkartusche nach einem der vorstehenden Ansprüche, **dadurch gekennzeichnet, dass** zwischen dem ersten Betätigungselement (34) und dem zweiten Betätigungselement (45) eine Betätigungssperre (54, 55) wirksam ist,

15. Messerkartusche nach einem der vorstehenden Ansprüche, **dadurch gekennzeichnet, dass** dem ersten Betätigungselement (34) ein Einsatzfühler (58) zugeordnet ist, der dazu eingerichtet ist, die Betätigung des ersten Betätigungselements (34) außerhalb des Instruments (12) zu sperren.

## Claims

1. Knife cartridge (23) for a surgical instrument (12),
having a cartridge housing (24) that is configured to be connectable with an instrument (12),
**characterized by**
a blade housing (31) that is movably supported inside the cartridge housing (24),
a blade (22) that is movably supported inside the blade housing (31),
a first operating element (34) for moving the blade housing (31) and
a second operating element (45) for moving the blade (22) inside the blade housing (31).

2. Knife cartridge according to claim 1, **characterized in that** the cartridge housing (24) comprises a latching means (27) for releasable connection of cartridge housing (24) with the instrument (12).

3. Knife cartridge according to claim 2, **characterized in that** the latching means (27) is operable by means of the first operating element (34).

4. Knife cartridge according to any of the preceding claims, **characterized in that** the blade housing (31) comprises a first locking position in which the blade housing (31) is completely positioned inside the cartridge housing (24).

5. Knife cartridge according to any of the preceding claims, **characterized in that** the blade housing (31) comprises a second locking position in which the blade housing is held partly projecting out of cartridge housing (24).

6. Knife cartridge according to any of the preceding claims, **characterized in that** the blade (22) comprises a retraction position in which the blade (22) is fully positioned inside the blade housing (31) and is locked inside the blade housing (31).

7. Knife cartridge according to any of the preceding claims, **characterized in that** the blade (22) comprises an extension (22a) that can be extended out of the blade housing (31).

8. Knife cartridge according to any of the preceding claims, **characterized in that** the first operating element (34) is movably supported on the cartridge housing (24).

9. Knife cartridge according to any of the preceding claims, **characterized in that** the first operating element (34) is supported on the cartridge housing (24) along a predefined movement path.

10. Knife cartridge according to claim 8, **characterized in that** a pivot lock (41) is effective between the first operating element (34) and cartridge housing (24) that is configured to allow a pivot movement of the first operating element (34) only in an end position of the movement path.

11. Knife cartridge according to any of the preceding claims, **characterized in that** the first operating element (34) comprises a coupling (39) for a connection with the blade housing (31).

12. Knife cartridge according to any of the preceding claims, **characterized in that** the first operating element (34) and the second operating element (45) can be alternatingly coupled with the blade (22) by means of a transfer mechanism (50).

13. Knife cartridge according to any of the preceding claims, **characterized in that** the second operating element (45) can be operatively coupled with the blade (22) by pivot movement of the first operating element (34).

14. Knife cartridge according to any of the preceding claims, **characterized in that** an operation lock (54, 55) is effective between the first operating element (34) and the second operating element (45).

15. Knife cartridge according to any of the preceding claims, **characterized in that** an insertion feeler (58) is assigned to the first operating element (34) that is configured to lock the operation of the first operating element (34) outside the instrument (12).

## Revendications

1. Cartouche de couteau (23) destinée à un instrument chirurgical (12),
comprenant un boîtier de cartouche (24) qui est réalisé de manière à pouvoir être relié à un instrument (12),
**caractérisée en ce qu'**elle comporte
un boîtier de lame (31) qui est monté de manière à pouvoir coulisser dans le boîtier de cartouche (24),
une lame (22) qui est montée de manière à pouvoir coulisser dans le boîtier de lame (31),
un premier élément d'actionnement (34) destiné au déplacement du boîtier de lame (31), et
un deuxième élément d'actionnement (45) destiné au déplacement de la lame (22) dans le boîtier de lame (31).

2. Cartouche de couteau selon la revendication 1, **caractérisée en ce que** le boîtier de cartouche (24) présente un moyen d'encliquetage (27) pour l'assemblage amovible du boîtier de cartouche (24) avec l'instrument (12).

3. Cartouche de couteau selon la revendication 2, **caractérisée en ce que** le moyen d'encliquetage (27) peut être actionné par le premier élément d'actionnement (34).

4. Cartouche de couteau selon l'une des revendications précédentes, **caractérisée en ce que** le boîtier de lame (31) présente une première position de blocage dans laquelle le boîtier de lame (31) est placé complètement à l'intérieur du boîtier de cartouche (24).

5. Cartouche de couteau selon l'une des revendications précédentes, **caractérisée en ce que** le boîtier de lame (31) présente une deuxième position de blocage dans laquelle le boîtier de lame (31) est tenu de manière à dépasser en partie du boîtier de cartouche (24).

6. Cartouche de couteau selon l'une des revendications précédentes, **caractérisée en ce que** la lame (22) présente une position rétractée dans laquelle la lame (22) est placée complètement à l'intérieur du boîtier de lame (31) et est verrouillée dans le boîtier de lame (31).

7. Cartouche de couteau selon l'une des revendications précédentes, **caractérisée en ce que** la lame (22) présente une saillie (22a) qui peut être coulissée pour sortir du boîtier de lame (31).

8. Cartouche de couteau selon l'une des revendications précédentes, **caractérisée en ce que** le premier élément d'actionnement (34) peut être monté de manière coulissante sur le boîtier de cartouche (24).

9. Cartouche de couteau selon l'une des revendications précédentes, **caractérisée en ce que** le premier élément d'actionnement (34) est monté sur le boîtier de cartouche (24), le long d'un chemin de déplacement prédéterminé.

10. Cartouche de couteau selon la revendication 8, **caractérisée en ce qu'**un moyen de blocage de pivotement (41) agit entre le premier élément d'actionnement (34) et le boîtier de cartouche (24) et est réalisé de manière à autoriser un pivotement du premier élément d'actionnement (34) uniquement dans une position d'extrémité du chemin de déplacement.

11. Cartouche de couteau selon l'une des revendications précédentes, **caractérisée en ce que** le premier élément d'actionnement (34) présente un accouplement (39) pour l'assemblage avec le boîtier de lame (31).

12. Cartouche de couteau selon l'une des revendications précédentes, **caractérisée en ce que** le premier élément d'actionnement (34) et le deuxième élément d'actionnement (45) peuvent être accouplés en alternance avec la lame (22), par l'intermédiaire d'un mécanisme de transfert (50).

13. Cartouche de couteau selon l'une des revendications précédentes, **caractérisée en ce que** le deuxième élément d'actionnement (45) peut être accouplé de manière opérationnelle avec la lame (22), par pivotement du premier élément d'actionnement (35).

14. Cartouche de couteau selon l'une des revendications précédentes, **caractérisée en ce qu'**un moyen de blocage d'actionnement (54, 55) agit entre le premier élément d'actionnement (34) et le deuxième élément d'actionnement (45).

15. Cartouche de couteau selon l'une des revendications précédentes, **caractérisée en ce qu'**un détecteur d'utilisation (58) est associé au premier élément d'actionnement (34) et est conçu pour bloquer l'actionnement du premier élément d'actionnement (34) à l'extérieur de l'instrument (12).
